(19) Europäisches Patentamt
European Patent Office
Office européen des brevets

(11) Numéro de publication : **0 101 386**
**B1**

(12) **FASCICULE DE BREVET EUROPÉEN**

(45) Date de publication du fascicule du brevet : **22.01.86**

(51) Int. Cl.4 : **C 07 D501/24**

(21) Numéro de dépôt : **83401654.5**

(22) Date de dépôt : **12.08.83**

(54) **Nouveaux dérivés de la céphalosporine et leur préparation.**

(30) Priorité : **13.08.82 FR 8214093**

(43) Date de publication de la demande : **22.02.84 Bulletin 84/08**

(45) Mention de la délivrance du brevet : **22.01.86 Bulletin 86/04**

(84) Etats contractants désignés : **AT BE CH DE FR GB IT LI LU NL SE**

(56) Documents cités :
**EP-A- 0 053 962**
**BE-A- 883 418**
**FR-A- 2 460 302**
**FR-A- 2 482 601**

(73) Titulaire : **RHONE-POULENC SANTE**
**Les Miroirs 18 Avenue d'Alsace**
**F-92400 Courbevoie Cedex (FR)**

(72) Inventeur : **Farge, Daniel**
**30 rue des Pins Sylvestres**
**F-94320 Thiais (FR)**
Inventeur : **Le Roy, Pierre**
**2 allée des Cerisiers**
**F-94320 Thiais (FR)**
Inventeur : **Moutonnier, Claude**
**3 rue Auguste Rodin**
**F-92350 Le Plessis-Robinson (FR)**
Inventeur : **Peyronel, Jean-François**
**36 parc d'Ardenay**
**F-91120 Palaiseau (FR)**
Inventeur : **Plau, Bernard**
**82 avenue Carnot**
**F-94100 Saint-Maur (FR)**

(74) Mandataire : **Gaumont, Robert et al**
**RHONE-POULENC RECHERCHES Service Brevets**
**Pharma 25, Quai Paul Doumer**
**F-92408 Courbevoie Cedex (FR)**

**EP 0 101 386 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux dérivés de la céphalosporine de formule générale :

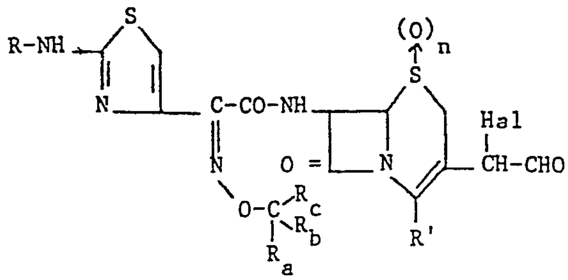

(I)

leurs formes isomères et leur mélange, et leur préparation.

Dans le brevet BE-A-883 418 et la demande de brevet EP-A-53 962, ont été décrites des céphalosporines portant en position – 3 un substituant formylméthyle.

Dans la formule générale (I),

— le symbole R représente un radical protecteur d'amino, facilement éliminable,

— le symbole R' représente un radical carboxy protégé,

— le symbole Hal représente un atome d'halogène (par exemple le chlore, le brome et l'iode),

— les symboles $R_a$, $R_b$ et $R_c$ représentent chacun un atome d'hydrogène ou bien $R_a$ représente un radical carboxy protégé et les symboles $R_b$ et $R_c$ sont identiques ou différents, et représentent des atomes d'hydrogène, ou des radicaux alcoyle contenant 1 à 4 atomes de carbone, ou forment ensemble un radical alcoylène contenant 2 à 5 atomes de carbone,

et n est égal à 0 ou 1.

Il est entendu que dans la formule générale (I) (et dans les formules générales employées ci-après) le groupement $—OCR_aR_bR_c$ se trouve en position syn.

Par ailleurs, les produits de formule générale (I), qui contiennent tous un radical —CH(Hal)—CHO en position – 3, présentent des formes isomères ; il est entendu que les épimères et leurs mélanges entrent dans le cadre de la présente invention.

De plus, lorsque dans la formule générale (I), les symboles $R_b$ et $R_c$ sont différents, il existe des diastéréoisomères ; il est entendu que ces formes isomères et leurs mélanges entrent dans le cadre de la présente invention.

Le radical protecteur d'amino R peut être par exemple choisi parmi les groupements t. butoxycarbonyle, trichloro-2,2,2 éthoxycarbonyle, trityle, benzyle, benzyloxycarbonyle, formyle, trifluoracétyle, p. nitrobenzyloxycarbonyle, p. méthoxybenzyloxycarbonyle ou encore diphénylphosphinoyle, ou un radical tel que défini par la formule générale

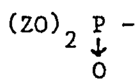

(II)

dans laquelle Z est alcoyle (contenant 1 à 4 atomes de carbone), trichloro-2,2,2 éthyle, phényle ou benzyle, ces deux derniers étant éventuellement substitués par un atome d'halogène ou par un radical alcoyle, alcoyloxy ou nitro, ou les symboles Z forment ensemble un radical alcoylène contenant 2 ou 3 atomes de carbone ; les radicaux phosphorés ci-dessus pouvant être introduits par application de la méthode décrite par A. MORIMOTO et coll., J. Chem. Soc. Perkin I 1109 (1980).

Le radical protecteur d'acide contenu par R' ou $R_a$ est un radical facilement éliminable, sans altération du reste de la molécule. A titre d'exemple, R' ou $R_a$ peuvent être protégés par un radical t. butyle, trichloro-2,2,2 éthyle, benzhydryle, p. nitrobenzyle ou p. méthoxybenzyle.

Selon l'invention, les dérivés de la céphalosporine de formule générale (I) pour lesquels n égale 0 peuvent être préparés par action d'un agent d'halogénation sur une énamine de formule générale :

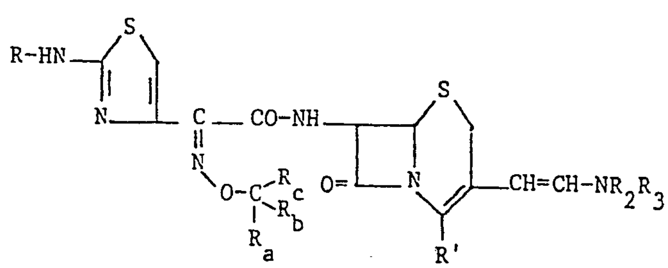

(III)

[dans laquelle R, R', $R_a$, $R_b$ et $R_c$ sont définis comme précédemment pour la formule générale (I), $R_2$ et $R_3$, identiques ou différents, représentent des radicaux alcoyle (éventuellement substitués par un radical alcoyloxy ou dialcoylamino) ou phényle, ou forment ensemble avec l'atome d'azote auquel ils sont rattachés, un hétérocycle à 5 ou 6 chaînons contenant éventuellement un autre hétéroatome choisi parmi l'azote, l'oxygène ou le soufre et éventuellement substitué par un radical alcoyle], suivie de l'hydrolyse du produit formé.

Par exemple on met en œuvre une énamine de formule générale (III) dans laquelle $R_2$ ou $R_3$ sont chacun un radical méthyle.

Parmi les agents d'halogénation peuvent être cités : les halogènes, les N-haloamides [par exemple N-bromo (ou N-chloro) succinimide, N-bromo (ou N-chloro) acétamide, dibromohydantoïne], les hypohalogénites d'alcoyle (par exemple hypochlorite d'éthyle ou de t. butyle, hypobromite de t. butyle).

On effectue généralement l'halogénation dans un solvant organique tel qu'un éther (par exemple tétrahydrofuranne, dioxanne), un solvant chloré (par exemple chlorure de méthylène, chloroforme), un ester (par exemple acétate d'éthyle), un alcool (par exemple méthanol, éthanol), un amide (par exemple diméthylformamide, diméthylacétamide), un nitrile (par exemple acétonitrile) ou une cétone (par exemple acétone), ou dans un mélange de tels solvants, à une température comprise entre − 70 et 0 °C.

L'hydrolyse s'effectue à une température comprise entre − 70 et 20 °C.

Selon l'invention, les produits de formule générale (I) dans laquelle n = 1 peuvent être obtenus par oxydation d'un produit de formule générale (I) dans laquelle n = 0, par toute méthode qui n'altère pas le reste de la molécule.

On opère notamment dans les conditions décrites dans la demande DE-A-2 637 176, notamment en présence d'acide m. chloroperbenzoïque dans un solvant organique tel que le dichlorométhane.

Les énamines de formule générale (III) peuvent être préparées selon la méthode décrite dans le brevet BE-A-883 416 ou dans la demande de brevet EP-A-53 961.

Les dérivés de la céphalosporine selon l'invention sont utiles pour la préparation de thiazolyl-3 céphalosporines de formule générale

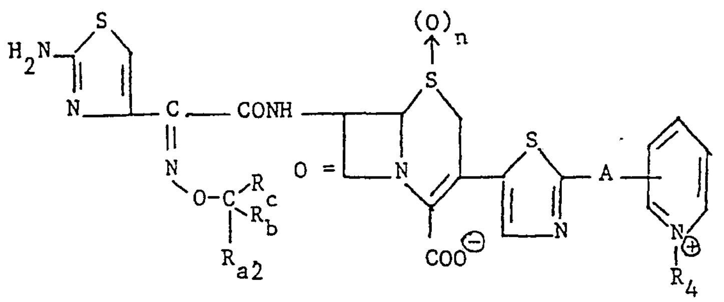

(IV)

dans laquelle, $R_b$, $R_c$ et n sont définis comme précédemment, le symbole $R_{a2}$ est un atome d'hydrogène ou représente un radical carboxy, le symbole A représente une liaison simple ou un radical bivalent choisi parmi $-CH_2-$, —NH— ou —NHCO— fixé en position − 3 ou − 4 du radical pyridinio, le symbole $R_4$ représente un radical méthyle, carboxyméthyle, carbamoylméthyle, benzyle ou allyle.

Il est entendu que les produits de formule générale (IV) existent soit sous forme de sel interne (l'un des groupements carboxy présents dans la molécule se trouvant à l'état de radical carboxylato), soit sous forme de solvat acide de cette bétaïne.

Les produits de formule générale (IV) peuvent être obtenus à partir des produits de formule générale (I) en opérant comme suit :

On fait agir un produit de formule générale

$$R_5 CS NH_2 \qquad (V)$$

dans laquelle $R_5$ est un radical amino ou un radical de structure

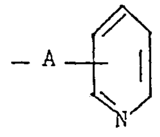

dans lequel A est défini comme précédemment, sur un dérivé de la céphalosporine de formule générale (I), puis éventuellement un agent de déshydratation, et réduit éventuellement le sulfoxyde obtenu et élimine éventuellement les radicaux protecteurs, pour obtenir un dérivé de la céphalosporine de formule générale

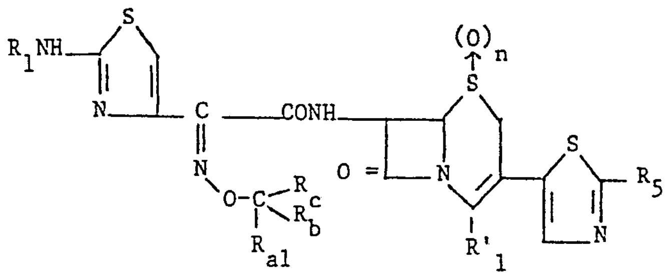

(VI)

dans laquelle $R_b$, $R_c$, $R_5$ et n sont définis comme précédemment, $R'_1$ est un radical carboxy libre ou protégé, $R_1$ est un atome d'hydrogène ou un radical protecteur d'amino et $R_{al}$ est un atome d'hydrogène ou un radical carboxy libre ou protégé.

On opère généralement en milieu organique ou hydroorganique, par exemple dans des solvants (ou des mélanges de solvants) tels que des alcools (méthanol, éthanol), des éthers (tétrahydrofuranne, dioxanne), des cétones (acétone), des nitriles (acétonitrile), des amides secondaires (diméthylformamide, diméthylacétamide), des esters (acétate d'éthyle) ou des acides (acide acétique, acide formique), en présence ou non d'une base (soude, potasse, carbonates, carbonates acides de métaux alcalins, sels d'acides carboxyliques et de métaux alcalins, amines tertiaires), à une température comprise entre − 50 °C et la température de reflux du mélange réactionnel.

Il est parfois préférable d'introduire un agent de déshydratation.

Parmi les agents de déshydratation utilisables, on peut citer : les halogénures d'acides sulfoniques [par exemple chlorure de tosyle, chlorure de méthanesulfonyle ou un halogénure du type $\underline{R}°SO_2Cl$ dans lequel $\underline{R}°$ est alcoyle, trifluoro (ou trichloro) méthyle ou phényle éventuellement substitué par halogène, méthyle ou nitro], les halogénures de phosphoryle (par exemple oxychlorure de phosphore) ou le chlorure de sulfonyle, soit dans un solvant basique [pyridine, amide (par exemple diméthylformamide, diméthylacétamide, hexaméthylphosphorotriamide)] soit dans un solvant chloré (par exemple chloroforme, chlorure de méthylène), un éther (par exemple tétrahydrofuranne), un ester, une cétone, un nitrile, ou un solvant aromatique, en présence d'une amine tertiaire (par exemple pyridine, quinoléine, triéthylamine).

La réduction du sulfoxyde s'effectue, le cas échéant, selon les méthodes décrites dans la demande DE-A-2 637 176.

L'élimination des groupements protecteurs d'acide peut s'effectuer par exemple :

— lorsqu'il s'agit d'un groupement t. butyle, p. méthoxybenzyle ou benzhydryle : par traitement en milieu acide, dans les conditions décrites ci-après pour l'élimination du radical trityle protecteur d'amino. Dans le cas du radical benzhydryle, on peut opérer en présence d'anisole ou par traitement par le chlorure d'aluminium dans les conditions décrites par T. Tsuji et coll., Tet. Lett., *30*, 2793 (1979) ;

— lorsqu'il s'agit d'un groupement trichloro-2,2,2 éthyle ou p. nitrobenzyle : par réduction (notamment traitement par le zinc dans l'acide acétique, ou lorsqu'il s'agit du groupement p. nitrobenzyle par hydrogénolyse).

L'élimination des groupements protecteurs d'amine peut s'effectuer par exemple :

— lorsqu'il s'agit d'un radical t. butoxycarbonyle, trityle, p. méthoxybenzyloxycarbonyle ou formyle : par traitement en milieu acide. De préférence on utilise l'acide trifluoracétique en opérant à une température comprise entre 0 et 20 °C, ou bien on utilise l'acide formique, phosphorique ou polyphosphorique anhydres ou aqueux à une température comprise entre 20 et 60 °C, ou encore l'acide paratoluènesulfonique ou méthanesulfonique dans l'acétone ou l'acétonitrile, à une température comprise entre 20 °C et la température de reflux du mélange réactionnel. Dans ces conditions le produit de formule générale (VI) peut être obtenu sous forme de trifluoracétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique ;

— lorsqu'il s'agit d'un radical trichloro-2,2,2 éthoxycarbonyle ou p. nitrobenzyloxycarbonyle ou d'un radical de formule générale (II) dans laquelle Z est trichloro-2,2,2 éthyle ou nitrobenzyle : par réduction (notamment traitement par le zinc dans l'acide acétique) ;

— lorsqu'il s'agit d'un radical benzyle ou benzyloxycarbonyle : par hydrogénation catalytique ;

— lorsqu'il s'agit d'un radical trifluoracétyle : par traitement en milieu basique ;

— lorsqu'il s'agit d'un radical de formule générale (II) : selon la méthode décrite dans le brevet belge 833 619 ;

— lorsqu'il s'agit d'un radical diphénylphosphinoyle : selon la méthode décrite par P. HAAKE et coll., J. Am. Chem. Soc., *95*, 8073 (1973).

Puis,

1) lorsque $R_5$ est un radical de structure

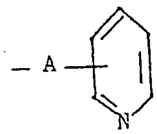

on fait agir un halogénure ou un sulfonate de formule générale

$$R_4—X \quad (VII)$$

dans laquelle R, est défini comme précédemment ou représente un radical carboxyméthyle protégé, et X représente un atome d'halogène choisi parmi l'iode, le brome ou le chlore, un reste alcoylsulfonyloxy [dont la partie alcoyle qui contient 1 à 4 atomes de carbone peut être substituée par 1 ou plusieurs atomes d'halogène] ou phénylsulfonyloxy [dont le radical phényle peut être substitué par un ou plusieurs substituants choisis parmi les atomes d'halogène ou les radicaux alcoyle ou nitro], sur un dérivé de la céphalosporine de formule générale (VI), puis éventuellement réduit le sulfoxyde obtenu, et élimine les radicaux protecteurs représentés par R ou contenus par $R_{al}$ ou $R'_1$.

La réaction s'effectue généralement dans un solvant organique tel qu'un amide (diméthylformamide, hexaméthylphosphorotriamide ou diméthylacétamide), un nitrile (acétonitrile par exemple), une cétone (acétone par exemple) ou un dérivé nitré (nitrométhane ou nitrobenzène par exemple), ou dans un mélange de tels solvants à une température comprise entre 0 et 80 °C.

Le cas échéant la réduction du sulfoxyde s'effectue selon les méthodes décrites dans la demande de brevet allemand 2 637 176.

L'élimination des radicaux protecteurs s'effectue dans les conditions décrites précédemment ; ou bien,

2) lorsque $R_5$ est un radical amino et lorsque l'on veut obtenir un produit de formule générale (IV) pour lequel le symbole A est un radical bivalent —NHCO—, on transforme le produit de formule générale (VI) obtenu, dans lequel $R_1$ est un radical protecteur d'amino et $R_5$ est un radical amino, par toute méthode connue pour former une fonction amide sans toucher au reste de la molécule.

La réaction s'effectue généralement par action d'un dérivé de l'acide nicotinique ou isonicotinique dont l'atome d'azote est quaternisé par un radical $R_4$ tel que défini précédemment étant entendu que lorsque $R_4$ est un radical carboxyméthyle, le radical carboxy est protégé.

On opère avantageusement par action d'un halogénure d'acide, puis on réduit éventuellement le sulfoxyde obtenu et élimine les radicaux protecteurs.

Lorsque l'on utilise le dérivé de l'acide nicotinique ou isonicotinique sous sa forme acide, on effectue la condensation sur l'(amino-2 thiazolyl-5)-3 céphalosporine de formule générale (VI) dans laquelle $R_1$ est un radical protecteur, en présence d'un agent de condensation tel qu'un carbodiimide (par exemple dicyclohexylcarbodiimide), le NN'-carbonyldiimidazole ou l'éthoxy-2 éthoxycarbonyl-1 dihydro-1,2 quinoléine, à une température comprise entre − 20 et 40 °C, puis on élimine les groupements protecteurs.

Lorsque l'on met en œuvre l'halogénure d'acide (qui peut être préparé in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone) ou dans des mélanges des solvants ci-dessus, en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine) ou en présence d'un agent de silylation tel que le bistriméthylsilylacétamide, ou bien un milieu hydroorganique en présence d'un agent alcalin de condensation tel que le bicarbonate de sodium, et l'on opère à une température comprise entre − 40 et 40 °C ; puis on élimine les groupements protecteurs dans les conditions décrites précédemment.

Les produits de formule générale (V) peuvent être préparés par action d'ammoniac sur l'isothiocyanate ou sur un dithiocarbamate d'alcoyle ou d'aryle correspondant, ou par action de sulfure d'hydrogène sur le nitrile correspondant, et notamment lorsque $R_5$ représente nicotinoylamino ou isonicotinoylamino, selon la méthode de W. H. PIKE, Chem. Ber., *6*, 755 (1873).

Les nouveaux produits selon la présente invention et les produits de formule générale (IV) peuvent être éventuellement purifiés par des méthodes physiques telles que la cristallisation, la chromatographie ou l'ultrafiltration.

Les dérivés de la céphalosporine de formule générale (IV) et leurs sels pharmaceutiquement acceptables sont des agents antibactériens à spectre large particulièrement intéressants. Ils manifestent une activité remarquable in vitro et in vivo sur les germes Gram-positifs et Gram-négatifs.

In vitro, les produits de formule générale (IV) se sont montrés actifs à une concentration comprise entre 1 et 30 μg/cm³ sur des souches de staphylocoques sensibles à la pénicilline G (Staphylococcus aureus Smith) et à une concentration comprise entre 0,01 et 1 μg/cm³ sur Escherichia coli souche NIHJ. De plus les produits de formule générale (IV) pour lesquels $R_a$ est un radical carboxy se sont montrés actifs à une concentration comprise entre 2 et 15 μg/cm³ sur Pseudomonas aeruginosa.

In vivo, les produits de formule générale (IV) se sont montrés actifs sur les infections expérimentales

de la souris à Staphylococcus aureus Smith (sensible à la pénicilline G) à une dose comprise entre 0,5 et 15 mg/kg par jour par voie sous-cutanée.

Par ailleurs, les produits de formule générale (IV) se sont montrés atoxiques à une dose de 1 mg/kg par voie sous-cutanée chez la souris.

Parmi les produits de formule générale (I), plus spécialement intéressants sont les produits pour lesquels le symbole R est un radical trityle, le symbole R′ est un radical benzhydryloxycarbonyle, le symbole Hal est un atome de brome et les symboles $R_a$,$R_b$ et $R_c$ représente chacun un atome d'hydrogène ou bien $R_a$ représente un radical t.butoxycarbonyle et $R_b$ et $R_c$ représentent des radicaux alcoyle, ainsi que leurs formes isomères et leurs mélanges.

Les exemples suivants, donnés à titre non limitatif, illustrent la présente invention.

## Exemple 1

A une solution de 1,72 g de benzhydryloxycarbonyl-2 (diméthylamino-2 vinyl)-3 [méthoxyimino-2 (tritylamino-2 thiazolyl-4)-2 acétamido]-7 oxo-8 thia-5 aza-1 bicyclo [4.2.0] octène-2 isomère syn forme E dans 30 $cm^3$ de tétrahydrofuranne sec refroidie à − 60 °C on ajoute, en 5 minutes, une solution de 0,1 $cm^3$ de brome dans 2 $cm^3$ de dichlorométhane. Le mélange réactionnel est agité 90 minutes à la même température puis versé dans un mélange de 150 $cm^3$ d'acétate d'éthyle et de 150 $cm^3$ d'eau distillée ; la phase organique est lavée 2 fois par 100 $cm^3$ d'eau distillée puis par 100 $cm^3$ de solution saturée de chlorure de sodium, séchée sur sulfate de sodium et concentrée à sec sous pression réduite (4 kPa) à 30 °C pour donner 1,75 g de benzhydryloxycarbonyl-2 (bromo-1 oxo-2 éthyl)-3 [méthoxyimino-2 (tritylamino-2 thiazolyl-4)-2 acétamido]-7 oxo-8 thia-5 aza-1 bicyclo [4.2.0] octène-2 isomère syn (mélange des deux diastéréoisomères au niveau du substituant en −3).

Spectre de RMN du proton (350 MHz, $CDCl_3$, δ en ppm, J en Hz) mélange de deux diastéréoisomères dans les proportions 50/50 3,35, 3,54, 3,66 et 3,75 (4d, J = 17,5, 2H, —S—$CH_2$— des deux diastéréoisomères) ; 4,08 (s, 3H, = N—$OCH_3$) ; 5,10 et 5,16 (2d, J = 5, 1H, —H en 6 des deux diastéréoisomères) ; 6 et 6,03 (2s, 1H, >CHBr des deux diastéréoisomères) ; 5,95 à 6,10 (mt, 1H, —H en 7 des deux diastéréoisomères) ; 6,76 (s, 1H, —H du thiazole) ; 6,91 et 6,98 (2s, 1H, —COO—C$\underline{H}$$(C_6H_5)_2$ des deux diastéréoisomères) ; 6,80 à 7,10 (mt, 2H, —CONH— et —NH—$C(C_6H_5)_3$ des deux diastéréoisomères) ; 7,20 à 7,60 (mt, aromatiques) ; 9,30 et 9,32 (2s, 1H, 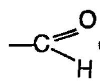 des deux diastéréoisomères).

## Exemple 2

Une solution de 0,6 g de benzhydryloxycarbonyl-2 [(t.butoxycarbonyl-2 propyl-2 oxyimino)-2 (tritylamino-2 thiazolyl-4)-2 acétamido]-7 (diméthylamino-2 vinyl)-3 oxo-8 thia-5 aza-1 bicyclo [4.2.0] octène-2 isomère syn forme E dans 10 $cm^3$ de tétrahydrofuranne est refroidie à − 60 °C. On ajoute une solution de 0,034 $cm^3$ de brome dans 1 $cm^3$ de dichlorométhane. Après agitation pendant 20 minutes à − 60 °C le mélange réactionnel est versé dans un mélange de 25 $cm^3$ d'acétate d'éthyle et de 50 $cm^3$ d'eau distillée. La phase organique est lavée 3 fois par 20 $cm^3$ d'eau distillée séchée et concentrée à sec sous pression réduite (4 kPa) à 30 °C pour donner 0,63 g de benzhydryloxycarbonyl-2 (bromo-1 oxo-2 éthyl)-3 [(t. butoxycarbonyl-2 propyl-2 oxyimino)-2 (tritylamino-2 thiazolyl-4)-2 acétamido]-7 oxo-8 thia-5-aza-1 bicyclo [4.2.0] octène-2 isomère syn (mélange des deux diastéréoisomères au niveau du substituant en − 3) sous la forme d'une meringue beige.

Spectre de RMN du proton (350 MHz, $CDCl_3$, δ en ppm, J en Hz) (signaux caractéristiques des deux diastéréoisomères A et B) 1,45 (s, 9H, —$C(CH_3)_3$ de A et B) ; 1,55 à 1,7 (4s, 4 × 3H, —$C(CH_3)_2$— de A et B) ; 5,10 et 5,15 (2d, J = 4, 2H, $H_6$ de A et B) ; 6,0 et 6,02 (2s, 2H, >CHBr de A et B) ; 6,73 (s large, 2H, H du thiazole de A et B) ; 6,88 et 6,94 (2s, 2H, —$CO_2C\underline{H}Ar_2$ de A et B) ; 9,28 et 9,3 (2s, 2H, —CHO de A et B).

## Exemple de référence 1

A une solution de 1,66 g de benzhydryloxycarbonyl-2 (bromo-1 oxo-2 éthyl)-3 [méthoxyimino-2 (tritylamino-2 thiazolyl-4)-2 acétamido]-7 oxo-8 thia-5 aza-1 bicyclo [4.2.0] octène-2 isomère syn (mélange des deux diastéréoisomères) dans 20 $cm^3$ de tétrahydrofuranne on ajoute 0,28 g de thionicotinamide et 0,16 g de pyridine puis on chauffe à 50 °C pendant 90 minutes. Le mélange réactionnel est ensuite refroidi à 0 °C, puis traité successivement par 0,23 g de chlorure de méthane sulfonyle et 0,56 $cm^3$ de triéthylamine. Après 30 minutes à 0 °C, le mélange réactionnel est versé sur un mélange de 200 $cm^3$ de solution saturée de bicarbonate de sodium et de 100 $cm^3$ d'acétate d'éthyle ; la phase organique est décantée, lavée par 150 $cm^3$ d'eau distillée puis par 150 $cm^3$ de solution saturée de chlorure de sodium, puis séchée sur sulfate de magnésium. Le résidu obtenu après évaporation à sec du solvant sous pression réduite (4 kPa) à 40 °C est chromatographié sur une colonne (hauteur : 30 cm, diamètre : 1,5 cm) de gel de silice (0,04-0,06 mm) en éluant sous une pression de 0,5 bar (50 kPa) par un mélange de cyclohexane et d'acétate d'éthyle (30/70 en volumes) et en recueillant des fractions de 50 $cm^3$. Les fractions 18 à 26 sont réunies et concentrées à sec sous pression réduite (30 mm de mercure ; 4 kPa) à 30 °C pour donner

0,18 g de benzhydryloxycarbonyl-2 [méthoxyimino-2 (trityl-amino-2 thiazolyl-4)-2 acétamido]-7 oxo-8 [(pyridyl-3)-2 thiazolyl-5]-3 thia-5 aza-1 bicyclo [4.2.0] octène-2 isomère syn sous la forme d'une poudre beige.

Spectre de RMN du proton (250 MHz, $CDCl_3$, δ en ppm, J en Hz) 3,60 et 3,76 (2d, J = 17,5, 2H, $—SCH_2—$) ; 4,11 (s, 3H, = $NOCH_3$) ; 5,18 (d, J = 4, 1H, H en 6) ; 6,04 (dd, J = 4 et 9, 1H, H en 7) ; 6,75 (s, 1H, H en 5 du thiazole) ; 6,96 (s, 1H, $—CO_2C\underline{H}Ar_2$) ; 7,0 à 7,4 (mf, 27H, aromatiques + —CONH— + H en 5 de la pyridine) ; 7,57 (s, 1H, H en 4 du thiazole) ; 8,04 (dd, 1H, H en 4 de la pyridine) ; 8,67 (dd, 1H, H en 6 de la pyridine) ; 8,94 (d, 1H, H en 2 de la pyridine).

On traite 1,1 g de benzhydryloxycarbonyl-2 [méthoxyimino-2 (tritylamino-2 thiazolyl-4)-2 acétamido]-7 oxo-8 [(pyridyl-3)-2 thiazolyl-5]-3 thia-5 aza-1 bicyclo [4.2.0] octène-2, isomère syn par 20 $cm^3$ d'acide formique, contenant 2 $cm^3$ d'anisole, pendant 30 minutes à 50 °C, puis concentre à sec le mélange réactionnel sous pression réduite (1 mm de mercure ; 0,13 kPa) à 40 °C. Le résidu est repris par 20 $cm^3$ d'éthanol que l'on évapore sous pression réduite (30 mm de mercure ; 4 kPa) à 40 °C ; cette opération est répétée encore 2 fois. Le résidu est concrété par 150 $cm^3$ d'éthanol. Le solide est essoré, lavé 2 fois par 5 $cm^3$ d'éthanol, 3 fois par 20 $cm^3$ d'éther éthylique et séché pour donner 0,56 g d'[(amino-2 thiazolyl-4)-2 méthoxyimino-2 acétamido]-7 carboxy-2 oxo-8 [(pyridyl-3)-2 thiazolyl-5]-3 thia-5 aza-1 bicyclo [4.2.0] octène-2 isomère syn sous la forme d'un solide jaune pâle.

Spectre infra-rouge (KBr), bandes caractéristiques ($cm^{-1}$) 3 380-3 320, 3 200, 3 100, 2 200, 1 780, 1 680, 1 620, 1 530, 1 040, 865, 810, 710.

Spectre de RMN du proton (250 MHz, DMSO $d_6$, δ en ppm, J en Hz) 3,87 (s, 3H, = $NOCH_3$) ; 32,88 et 4,0 (2d, J = 17, 2H, $—SCH_2—$) 5,28 (d, J = 5, 1H, H en 6) ; 5,9 (dd, J = 5 et 8, 1H, H en 7) ; 6,77 (s, 1H, H en 5 du thiazole) ; 7,22 (s, 2H, $—NH_2$), 7,55 (dd, J = 8 et 5, 1H, H en 5 de la pyridine) ; 8,02 (s, 1H, H en 4 du thiazole) ; 8,28 (m, 1H, H en 4 de la pyridine) ; 8,68 (dd, J = 5 et 1,5, 1H, H en 6 de la pyridine) ; 9,11 (d, J = 1,5, 1H, H en 2 de la pyridine ; 14,5 à 12 (mf étalé, 1H, $—COO\underline{H}$).

On agite à 25 °C pendant 24 heures un mélange de 0,06 g d'[(amino-2 thiazolyl-4)-2 méthoxyimino-2 acétamido]-7 carboxy-2 oxo-8 [(pyridyl-3)-2 thiazolyl-5]-3 thia-5 aza-1 bicyclo [4.2.0] octène-2 isomère syn, avec 0,04 $cm^3$ d'iodure de méthyle dans 0,5 $cm^3$ de NN diméthylformamide. Le mélange réactionnel est dilué par 10 $cm^3$ d'acétate d'éthyle ; un solide précipite, il est filtré, lavé par 2 fois 2 $cm^3$ d'acétate d'éthyle puis 2 fois 5 $cm^3$ d'éther éthylique et séché sous pression réduite (0,1 mm de mercure ; 0,013 kPa) à 25 °C pour donner 0,06 g d'iodhydrate d'[(amino-2 thiazolyl-4)-2 méthoxyimino-2 acétamido]-7 carboxylato-2 [(méthyl-1 pyridinio-3)-2 thiazolyl-5]-3 oxo-8 thia-5 aza-1 bicyclo [4.2.0] octène-2 brut sous la forme d'un solide jaune.

Spectre de RMN du proton (250 MHz, DMSO $d_6$, δ en ppm, J en Hz) 3,91 (s, 3H, = $NOCH_3$) ; 3,99 (s large, 2H, $—SCH_2—$) ; 4,46 (s, 3H, $N^{\oplus}—CH_3$) ;
⩾5,31 (d, J = 5, 1H, H en 6) ; 5,99 (dd, J = 5 et 9, 1H, H en 7) ; 6,89 (s, 1H, H en 4 du thiazole) ; 8,25 (s, 1H, H en 5 du thiazole) ; 8,27 (mf, 1H, H en 5 du pyridinio) ; 9,05 (d, 1H, H en 4 du pyridinio) ; 9,14 (mf, 1H, H en 6 du pyridinio) ; 9,69 (s, 1H, H en 2 du pyridinio) ; 9,86 (d, J = 9, 1H, —CONH—).

7,6 g d'iodhydrate d'[(amino-2 thiazolyl-4)-2 méthoxyimino-2 acétamido]-7 carboxylato-2 [(méthyl-1 pyridinio-3)-2 thiazolyl-5]-3 oxo-8 thia-5 aza-1 bicyclo [4.2.0] octène-2 isomère syn brut obtenus dans des conditions identiques à celles décrites ci-dessus, sont repris par 500 $cm^3$ d'eau distillée et 250 $cm^3$ d'acétate d'éthyle ; après filtration et décantation de la phase aqueuse, celle-ci est lavée par 150 $cm^3$ d'acétate d'éthyle et traitée par de la résine IR 45 basique jusqu'à atteindre un pH de 4,7. La résine est éliminée par filtration et la solution aqueuse est lyophilisée. Le lyophilisat est concrété par 50 $cm^3$ d'eau distillée pour donner 3,15 g d'[(amino-2 thiazolyl-4)-2 méthoxyimino-2 acétamido]-7 carboxylato-2 [(méthyl-1 pyridinio-3)-2 thiazolyl-5]-3 oxo-8 thia-5 aza-1 bicyclo [4.2.0] octène-2 isomère syn sous la forme d'une poudre orangée.

Spectre infra-rouge (KBr, bandes caractéristiques en $cm^{-1}$) 3 600-2 500, 1 765, 1 670, 1 610, 1 530, 1 380, 1 030, 770, 675.

Spectre RMN du proton (250 MHz, DMSO $d_6$, δ en ppm, J en Hz) 3,71 et 3,84 (2D, J = 17, 2H,

$—S—CH_2—$) ; 3,86 (S, 3H, = $N—OCH_3$) ; 4,43 (S, 3H, $\geqslant \overset{+}{N}—CH_3$) ;

5,15 (D, J = 5, 1H, —H en 6) ; 5,65 (DD, J = 8 et 5, 1H, —H en 7) ; 6,76 (S, 1H, —H en 5 du thiazole) ; 7,27 (Mf, 2H, $—NH_2$) ; 8,08 (S, 1H, —H en 4 du thiazole) ; 8,14 (DD, J = 8 et 6, 1H, —H en 5 du pyridinio) ; 8,83 (D, J = 8, 1H, —H en 4 du pyridinio) ; 8,98 (D, J = 6, 1H, —H en 6 du pyridinio ; 9,48 (S, 1H, —H en 2 du pyridinio) ; 9,64 (D, J = 8, 1H, —CONH—).

Exemple de référence 2

Un mélange de 0,08 g de (pyridyl-3) thiourée et de 0,55 g de benzhydryloxycarbonyl-2(bromo-1 oxo-2 éthyl)-3 [(t.butoxycarbonyl-2 propyl-2 oxyimino)-2 (tritylamino-2 thiazolyl-4)-2 acétamido]-7 oxo-8 thia-5 aza-1 bicyclo [4.2.0] octène-2 isomère syn (mélange des deux diastéréoisomères au niveau du substituant en −3) dans 10 $cm^3$ de tétrahydrofuranne est agité 35 minutes à 25 °C, puis additionné de 0,042 $cm^3$ de pyridine et de 1 $cm^3$ d'eau. Le mélange réactionnel est agité 16 heures à 25 °C, puis versé dans un

mélange de 50 $cm^3$ d'acétate d'éthyle, et de 50 $cm^3$ d'acide chlorhydrique 0,2 N. La solution organique est lavée par 50 $cm^3$ de solution saturée de bicarbonate de sodium, 3 fois par 50 $cm^3$ d'eau puis par 20 $cm^3$ de solution saturée de chlorure de sodium et séchée sur sulfate de magnésium. Le résidu obtenu après évaporation à sec du solvant sous pression réduite (4 kPa) à 30 °C est chromatographié sur une colonne (hauteur : 24 cm, diamètre : 2 cm) de gel de silice (0,04-0,06 mm) en éluant sous une pression de 0,5 bar (50 kPa) par un mélange de cyclohexane et d'acétate d'éthyle (15-85 en volumes) et en recueillant des fractions de 15 $cm^3$. Les fractions 7 à 13 sont réunies et concentrées à sec sous pression réduite (4 kPa) à 30 °C pour donner 0,39 g de benzhydryloxycarbonyl-2 [(t.butoxycarbonyl-2 propyl-2 oxyimino)-2 (tritylamino-2 thiazolyl-4)-2 acétamido]-7 oxo-8 [(pyridyl-3 amino)-2 thiazolyl-5]-3 thia-5 aza-1 bicyclo [4.2.0] octène-2 isomère syn sous la forme d'une meringue jaune.

Spectre infra-rouge ($CHBr_3$), bandes caractéristiques ($cm^{-1}$) 3 400, 1 790, 1 725, 1 680, 1 525, 1 495, 1 450, 1 370, 750, 740.

Spectre de RMN du proton (250 MHz, DMSO $d_6$ δ en ppm, J en Hz) 1,37 (S, 9H, —$C(CH_3)_3$) ; 1,43 (S, 6H, >$C(CH_3)_2$) ; 3,77 et 3,89 (2D, J = 17,5, 2H, —S—$CH_2$—) ; 5,28 (D, J = 5, 1H, —H en 6) ; 5,83 (DD, J = 8 et 5, 1H, —H en 7) ; 6,72 (S, 1H, —H en 5 du thiazole) ; 6,89 (S, 1H, —COO—$\underline{C}H(C_6H_5)_2$) ; 7 à 7,4 (Mt, aromatiques, —H en 4 du thiazole et —H en 5 de la pyridine) ; 8,07 (D large, J = 7,5, 1H, —H en 4 de la pyridine) ; 8,19 (D large, J = 5, 1H, —H en 6 de la pyridine) ; 8,68 (D, J = 2, 1H, —H en 2 de la pyridine) ; 8,83 (S, 1H, —$\underline{N}H$—$C(C_6H_5)_3$) ; 9,48 (D, J = 8, 1H, —CO—NH—) ; 10,44 (S, 1H, >N—H).

A une solution refroidie à 0 °C de 13,46 g de benzhydryloxycarbonyl-2{[(t.butoxycarbonyl-2 propyl-2)oxyimino]-2 (tritylamino-2 thiazolyl-4)-2 acétamido}-7 oxo-8 [(pyridyl-3 amino)-2 thiazolyl-5]-3 thia-5 aza-1 bicyclo [4.2.0] octène-2, isomère syn dans 123 $cm^3$ de dichlorométhane, on coule pendant 30 minutes une solution de 2,59 g d'acide m-chloroperbenzoïque dans 52 $cm^3$ de chlorure de méthylène. On agite pendant 15 minutes à 0 °C puis on dilue la solution réactionnelle par 200 $cm^3$ de chlorure de méthylène. Le mélange est lavé successivement par 200 $cm^3$ d'une solution demi-saturée de bicarbonate de sodium, puis deux fois 200 $cm^3$ d'eau distillée. La phase organique est alors séchée sur sulfate de magnésium anhydre, filtrée puis concentrée sous pression réduite (100 mm de mercure, 13,3 kPa) à 30 °C. Le résidu est purifié par chromatographie sur une colonne (hauteur = 29 cm, diamètre = 5,8 cm) de gel de silice (0,04-0,06 mm) en éluant sous une pression de 0,4 bar par 2 litres d'un mélange cyclohexane-acétate d'éthyle (10/90 en volumes) et en recueillant des fractions de 125 $cm^3$. Les fractions 11 à 15 sont réunies et concentrées à sec sous pression réduite (30 mm de mercure ; 4 kPa) à 30 °C pour donner 0,70 g de benzhydryloxycarbonyl-2 {[(t.butoxycarbonyl-2 propyl-2)oxyimino]-2 (tritylamino-2 thiazolyl-4)-2 acétamido}-7 oxo-8 oxyde-5 [(pyridyl-3 amino)-2 thiazolyl-5]-3 thia-5 aza-1 bicyclo [4.2.0] octène-2, isomère syn, sous la forme d'une meringue jaune.

Spectre infra-rouge ($CHBr_3$), bandes caractéristiques ($cm^{-1}$) 3 390, 1 800, 1 725, 1 680, 1 530, 1 495, 1 450, 1 370, 1 050, 700.

Spectre de RMN du proton (250 MHz, $CDCl_3$, δ en ppm, J en Hz) 1,41 (S, 9H, —$C(CH_3)_3$) ; 1,56 et 1,58 (2S, 6H, >$C(CH_3)_2$) ; 3,25 et 3,84 (2D, J = 18,5, 2H, —S—$CH_2$—) ; 4,56 (D, J = 5, 1H, —H en 6) ; 6,19 (DD, J = 9,5 et 5, 1H, —H en 7) ; 6,72 (S, 1H, —H en 5 du thiazole) ; 6,92 (S, 1H, —COO—$C\underline{H}(C_6H_5)_2$) ; 7,01 (S, 1H, —H en 4 du thiazole) ; 7,11 (DD, J = 5 et 7,5, —H en 5 de la pyridine) ; 7,05 à 7,45 (Mt, aromatiques) ; 7,9 (DDD, J = 7,5, 2 et 1,5, 1H, —H en 4 de la pyridine) ; 8,0 (D, J = 9,5, 1H, —CONH—) ; 8,21 (DD, J = 5 et 1,5, 1H, —H en 6 de la pyridine) ; 8,6 (D, J = 2, 1H, —H en 2 de la pyridine) : 8,73 (Mf, 1H, —$N\underline{H}C(C_6H_5)_3$).

Une solution de 1,67 g de benzhydryloxycarbonyl-2{[(t.butoxycarbonyl-2 propyl-2)oxyimino]-2 (tritylamino-2 thiazolyl-4)-2 acétamido]-7 oxo-8 oxyde-5 [(pyridyl-3 amino)-2 thiazolyl-5]-3 thia-5 aza-1 bicyclo [4.2.0] octène-2, isomère syn, et de 0,10 $cm^3$ d'iodure de méthyle dans 15 $cm^3$ de N,N-diméthylformamide est agitée pendant 24 heures. On dilue le mélange en ajoutant 30 $cm^3$ d'oxyde d'isopropyle. Les liqueurs surnageantes sont décantées, et le résidu est agité avec 50 $cm^3$ d'oxyde d'éthyle. Le précipité est filtré et séché sous pression réduite (0,1 mm de mercure, 0,013 kPa) à 20 °C. On obtient 1,53 g d'iodure de benzhydryloxycarbonyl-2{[(t.butoxycarbonyl-2 propyl-2) oxyimino]-2 (tritylamino-2 thiazolyl-4)-2 acétamido}-7 [(méthyl-1 pyridinio-3 amino)-2 thiazolyl-5]-3 oxo-8 oxyde-5 thia-5 aza-1 bicyclo [4.2.0] octène-2, isomère syn.

Spectre infra-rouge ($CHBr_3$), bandes caractéristiques ($cm^{-1}$) 3 380, 3 100, 2 500, 1 806, 1 730, 1 675, 1 600, 1 505, 1 450, 1 370, 1 145, 755, 700, 675.

Spectre de RMN du proton (250 Mhz, DMSO $d_6$, δ en ppm, J en Hz) 1,36 (S, 9H, —$C(CH_3)_3$) ; 1,44 et 1,45 (2S, 6H, >$C(CH_3)_2$) ; 3,83 et 4,37 (2D, J = 18,5, 2H, —$SCH_2$—) ; 4,39 (S, 3H, $\overset{+}{\geqslant}$N—$CH_3$) ; 5,12 (D, J = 5, 1H, —H en 6) ; 6,08, DD, J = 9 et 5, 1H, —H en 7 ; 6,80 (S, 1H, —H en 5 du thiazole) ; 6,95 (S, 1H, —COO—$C\underline{H}(C_6H_5)_2$) ; 7 à 7,4 (Mt, aromatiques et —H en 4 du thiazole) ; 8,05 (DD, J = 8 et 6, 1H, —H en 5 du pyridinio) ; 8,35 (D, J = 9, 1H, —CO—NH—) ; 8,41 (D large, J = 8, 1H, —H en 4 du pyridinio) ; 8,6 (D, J = 6, 1H, —H en 6 du pyridinio) ; 8,78 (S, 1H, —$N\underline{H}C(C_6H_5)_3$) ; 9,19 (S large, 1H, —H en 2 du pyridinio) ; 11,33 (Mf, 1H, >NH).

A une solution refroidie à 5 °C de 1,25 g de benzhydryloxycarbonyl-2 {[(t. butoxycarbonyl-2 propyl-2)oxyimino)-2 (tritylamino-2 thiazolyl-4)-2 acétamido}-7 [(méthyl-1 pyridinio-3 amino)- thiazolyl-5]-3 oxo-8 oxyde-5 thia-5 aza-1 bicyclo [4.2.0] octène-2, isomère syn dans 1 $cm^3$ d'anisole, on ajoute 6,5 $cm^3$ d'acide trifluoracétique et 0,65 $cm^3$ d'eau distillée. On agite à cette température pendant 10 minutes puis 1 heure

15 minutes à 22 °C. Le mélange réactionnel est ensuite dilué dans 4,3 cm$^3$ d'acétone puis 40 cm$^3$ d'oxyde d'éthyle. On agite pendant 5 minutes, filtre et lave le précipité par 5 fois 10 cm$^3$ d'oxyde d'éthyle. On obtient 0,78 g de ditrifluoracétate d'{[(amino-2 thiazolyl-4)-2 [(carboxy-2 propyl-2) oxyimino]-2 acétamido}-7 carboxy-2 [(méthyl-1 pyridinio-3 amino)-2 thiazolyl-5]-3 oxo-8 oxyde-5 thia-5 aza-1 bicyclo [4.2.0] octène-2, isomère syn, sous la forme d'une poudre brun verdâtre.

Spectre infra-rouge (KBr), bandes caractéristiques (cm$^{-1}$) 3 700, 2 200, 1 790, 1 675, 1 635, 1 525, 1 510, 1 200, 1 145, 800, 720, 670.

Spectre de RMN du proton (250 MHz, DMSO $d_6$, δ en ppm, J en Hz) 1,51 et1,52 (2S, 6H, $>C(CH_3)_2$) ; 3,8 et 4,41(2D, J = 18,5, 2H, —S—CH$_2$—) ; 4,36 (S, 3H, $>\overset{+}{N}-CH_3$) ; 5,11 (D, J = 5, 1H, —H en 6) ; 6,08 (DD, J = 8 et 5, 1H, —H en 7) ; 6,86 (S, 1H, —H en 5 du thiazole) ; 7,10 à 7,8 (Mf, 3H, —$\overset{+}{N}H_3$) ; 7,54 (S, 1H, —H en 4 du thiazole) ; 8,01 (DD, J = 8,5 et 5, 1H, —H en 5 du pyridinio) ; 8,44 (D large, J = 8,5, 1H, —H en 4 du pyridinio) ; 8,56 (D, J = 5, 1H, —H en 6 du pyridinio) ; 8,63 (D, J = 8, 1H, —CONH—) ; 9,35 (S large, 1H, —H en 2 du pyridinio) ; 11,6 (Mf, 1H, $>$N—H) ; 14 à 11 (Mf étalé, —COOH).

A une suspension de 0,71 g de ditrifluoracétate d'{[(amino-2 thiazolyl-4)-2 [(carboxy-2 propyl-2)oxyimino]-2 acétamido}-7 carboxy-2 [(méthyl-1 pyridinio-3 amino)-2 thiazolyl-5]-3 oxo-8 oxyde-5 thia-5 aza-1 bicyclo [4.2.0] octène-2, isomère syn dans 21,3 cm$^3$ d'eau distillée, on ajoute une solution de 1,75 cm$^3$ de résine Amberlite LA-2 dans 5,35 cm$^3$ de méthylisobutylcétone. Le mélange est agité jusqu'à pH 6,2 puis la phase liquide est filtrée et le filtrat est lyophilisé. Le lyophilisat est agité pendant 2 heures dans 25 cm$^3$ d'éther anhydre, filtré et lavé par 2 fois 10 cm$^3$ d'éther, séché sous pression réduite(0,1 mm de mercure, 0,013 kPa) pour donner 0,12 g d'{(amino-2 thiazolyl-4)-2 [(carboxy-2 propyl-2)oxyimino]-2 acétamido}-7 carboxylato-2 [(méthyl-1 pyridinio-3 amino)-2 thiazolyl-5]-3 oxo-8 oxyde-5 thia-5 aza-1 bicyclo [4.2.0] octène-2, isomère syn, sous la forme d'une poudre jaune.

Spectre infra-rouge (KBr), bandes caractéristiques (cm$^{-1}$) 1 780, 1 670, 1 610, 1 530, 1 395, 1 040, 675.

Spectre de RMN du proton (250 MHz, DMSO $d_6$, δ en ppm, J en Hz) vers 1,51 ppm (Mf, $>C(CH_3)_2$) ; 3,78 et 4,25 (2D, J = 18,5, 2H, —S—CH$_2$—) ; 4,31 (S, 3H, $>\overset{+}{N}-CH_3$) ; 5,05 (D, J = 4, 1H, —H en 6) ; 5,9 (MF, 1H, —H en 7) ; 6,81 (S, 1H, —H en 5 du thiazole) ; 7,23 (Mf, 2H, —NH$_2$) ; 7,49 (S, 1H, —H en 4 du thiazole) ; 7,87 (Mf, 1H, —H en 5 du pyridinio) ; 8,42 (Mf, 1H, —H en 6 du pyridinio) ; 9,05 (Mf, 1H, H en 4 du pyridinio) ; 5,20 (Mf, 1H, —H en 2 du pyridinio).

## Exemple de référence 3

L'iodure de benzhydryloxycarbonyl-2 [(t. butoxycarbonyl-2 propyl-2)oxyimino-2 (tritylamino-2 thiazolyl-4)-2 acétamido]-7 [(méthyl-1 pyridinio-3 amino)-2 thiazolyl-5]-3 oxo-8 oxyde-5 thia-5 aza-1 bicyclo [4.2.0] octène-2, isomère syn obtenu comme à l'exemple de référence 2 peut être traité de la manière suivante :

A une solution refroidie à 0 °C de 3,76 g d'iodure de benzhydryloxycarbonyl-2 [(t.butoxycarbonyl-2 propyl-2)oxyimino-2 (tritylamino-2 thiazolyl-4)-2 acétamido]-7 [méthyl-1 pyridinio-3 amino)-2 thiazolyl-5]-3 oxo-8 oxyde-5 thia-5 aza-1 bicyclo [4.2.0] octène-2, isomère syn dans 30 cm$^3$ de chlorure de méthylène, on ajoute 2,4 cm$^3$ de N,N-diméthylacétamide, puis 1,06 cm$^3$ de trichlorure de phosphore. On agite pendant 45 minutes à 0 °C puis on dilue la masse réactionnelle dans 120 cm$^3$ d'acétate d'éthyle. Un produit précipite, on filtre le précipité et on le lave par 3 fois 25 cm$^3$ d'acétate d'éthyle, et 2 fois 25 cm$^3$ d'éther éthylique. Le produit est dissous dans 100 cm$^3$ de chlorure de méthylène, puis traité au noir 3S. Le filtrat est concentré à sec sous pression réduite (100 mm de mercure ; 13,3 kPa) et le résidu est repris par 50 cm$^3$ d'éther éthylique. Le mélange hétérogène est filtré et le solide lavé par 3 fois 15 cm$^3$ d'éther éthylique. Il est séché sous pression réduite (0,1 mm de mercure ; 0,013 kPa) et on obtient 2,95 g d'iodure de benzhydryloxycarbonyl-2 [(t. butoxycarbonyl-2 propyl-2)oxyimino-2 (tritylamino-2 thiazolyl-4)-2 acétamido]-7 [(méthyl-1 pyridinio-3 amino)-2 thiazolyl-5]-3 oxo-8 thia-5 aza-1 bicyclo [4.2.0] octène-2 isomère syn.

Spectre infra-rouge (KBr), bandes caractéristiques (cm$^{-1}$) 2 980, 2 940, 1 790, 1 728, 1 698, 1 595, 1 575, 1 530, 1 510, 1 450, 1 375, 1 225, 1 142, 1 003, 760, 705.

Spectre de RMN du proton (250 MHz, DMSO $d_6$, δ en ppm, J en Hz) 1,38 (S, 9H, —COO—C(CH$_3$)$_3$) ; 1,42 (S, 6H, $>C(CH_3)_2$) ; 3,79 et 3,94 (2D, J = 17,5, 2H, —S—CH$_2$—) ; 4,38 (S, 3H, $>\overset{+}{N}-CH_3$) ; 5,27 (D, J = 5, 1H, —H en 6) ; 5,85 (DD, J = 8 et 5, 1H, —H en 7) ; 6,72 (S, 1H, —H en 5 du thiazole) ; 6,90 (S, 1H, —COO—$\underline{CH}(C_6H_5)_2$) ; 6,95 à 7,4 (Mt, aromatiques + H en 4 du thiazole) ; 8,05 (DD, J = 8 et 5,5, 1H, —H en 5 du pyridinio) ; 8,39 (D, J = 8, 1H, —H en 4 du pyridinio) ; 8,59 (D, J= 5,5, 1H, —H en 6 du pyridinio) ; 8,83 (S,1H, —$\underline{NH}$—C(C$_6$H$_5$)$_3$) ; 9,16 (S large, 1H, —H en 2 du pyridinio) ; 9,48 (D, J = 8, 1H, —CO—NH—) ; 11,29 (Mf, 1H, —NH—).

On chauffe à 50 °C pendant 30 minutes une solution de 2,85 g d'iodure de benzhydryloxycarbonyl-2 [(t.butoxycarbonyl-2 propyl-2)oxyimino-2 (tritylamino-2 thiazolyl-4)-2 acétamido]-7 [(méthyl-1 pyridinio-3 amino)-2 thiazolyl-5]-3 oxo-8 thia-5 aza-1 bicyclo [4.2.0] octène-2, isomère syn dans 42,8 $cm^3$ d'acide formique et 4,3 $cm^3$ d'anisole. Le mélange est alors dilué par 14,3 $cm^3$ d'eau distillée et agité à 50 °C pendant 15 minutes. La solution réactionnelle est concentrée à sec sous pression réduite (0,1 mm de mercure ; 0,013 kPa) à 40 °C. Le résidu est repris dans 100 $cm^3$ d'éthanol que l'on évapore sous pression réduite (0,1 mm de mercure ; 0,013 kPa) jusqu'à un volume résiduel d'environ 40 $cm^3$. On répète l'opération et on filtre le produit que l'on lave par 2 fois 25 $cm^3$ d'éthanol, 2 fois 25 $cm^3$ d'acétate d'éthyle et 2 fois 25 $cm^3$ d'éther éthylique. Le produit (0,95 g) est séché sous pression réduite (0,1 mm de mercure ; 0,013 kPa). On en prélève 0,58 g que l'on dissout dans 0,75 $cm^3$ d'anisole. On obtient une solution marron que l'on refroidit vers 5 °C. On ajoute une solution froide de 4,90 $cm^3$ d'acide trifluoracétique et 0,49 $cm^3$ d'eau distillée. Le mélange est agité 15 minutes à 5 °C puis 1 heure 15 minutes à 25 °C. Le mélange réactionnel est dilué par 3,2 $cm^3$ d'acétone, puis on coule 13 $cm^3$ d'éther éthylique. Le mélange est agité pendant 10 minutes, filtré et le précipité est lavé par 2 fois 10 $cm^3$ d'éther éthylique et séché sous pression réduite (0,1 mm de mercure, 0,013 kPa). On obtient 0,60 g de ditrifluoracétate d'{[(amino-2thiazolyl-4)-2 [(carboxy-2 propyl-2)oxyimino]-2 acétamido}-7 carboxy-2 [(méthyl-1 pyridinio-3 amino)-2 thiazolyl-5]-3 oxo-8 thia-5 aza-1 bicyclo [4.2.0] octène-2, isomère syn, sous la forme d'une poudre marron clair.

A 0,62 g de ditrifluoracétate d'{[(amino-2 thiazolyl-4)-2 [(carboxy-2 propyl-2) oxyimino]-2 acétamido}-7 carboxy-2 [(méthyl-1 pyridinio-3 amino)-2 thiazolyl-5]-3 oxo-8 thia-5 aza-1 bicyclo [4.2.0] octène-2, isomère syn dans 62 $cm^3$ d'eau distillée, on ajoute 12 $cm^3$ de résine Amberlite IR 45 (OH). On agite le mélange pendant 40 minutes, on filtre la résine et on extrait le filtrat par 25 $cm^3$ d'acétate d'éthyle. La phase aqueuse est concentrée à l'évaporateur rotatif sous pression réduite (30 mm de mercure ; 4 kPa) jusqu'à un volume résiduel de 50 $cm^3$. Elle est alors lyophilisée pour donner 0,15 g d'un lyophilisat jaune pâle d'{(amino-2 thiazolyl-4)-2 [(carboxy-2 propyl-2)oxyimino]-2 acétamido}-7 carboxylato-2 [(méthyl-1 pyridinio-3 amino)-2 thiazolyl-5]-3 oxo-8 thia-5 aza-1 bicyclo [4.2.0] octène-2, isomère syn.

Spectre infra-rouge (KBr), bandes caractéristiques ($cm^{-1}$) 3 420, 3 100, 2 985, 1 765, 1 670, 1 600, 1 575, 1 525, 1 470, 1 390, 1 360, 1 335, 1 290, 1 220, 1 150, 1 075, 1 030, 980, 910, 830, 810, 765, 670, 610, 570, 530, 430, 375.

Spectre de RMN du proton (250 MHz, DMSO $d_6$, δ en ppm, J en Hz) 1,47 et 1,50 (2S, 6H, $>C(CH_3)_2$) ; 3,75 et 3,90 (2D, J = 17,5, 2H, —S—$CH_2$—) ; 4,30 (S large, 3H, $\geqslant \overset{}{\underset{+}{N}}CH_3$) ;

5,23 (D, J = 4, 1H, —H en 6) ; 5,78 (DD, J = 4 et 8, 1H, —H en 7) ; 6,76 (S, 1H, —H en 5 du thiazole) ; 7,26 (S large, 2H, —$NH_2$) ; 7,50 (S, 1H, —H en 5 du thiazole) ; 7,83 (Mf, 1H, H en 5 du pyridinio) ; 8,35 (D, 1H, H en 6 du pyridinio) ; 9,02 (D, 1H, H en 4 du pyridinio) ; 9,17 (S large, 1H, —H en 2 du pyridinio).

**Revendications** (pour les Etats Contractants : BE, CH, DE, FR, GB, IT, LI, LU, NL, SE)

1. Un dérivé de la céphalosporine caractérisé en ce qu'il répond à la formule générale

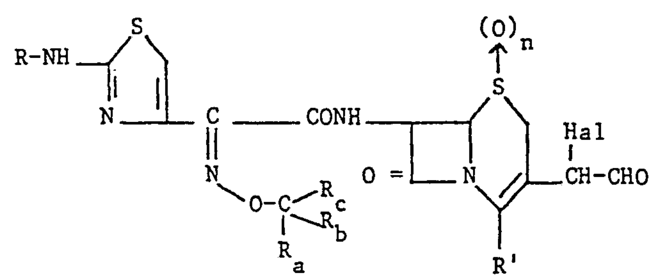

de forme syn, dans laquelle

le symbole R représente un radical protecteur d'amino,

le symbole R' représente un radical carboxy protégé,

le symbole Hal représente un atome d'halogène,

les symboles $R_a$, $R_b$ et $R_c$ représentent chacun un atome d'hydrogène, ou bien $R_a$ représente un radical carboxy protégé et, $R_b$ et $R_c$ qui sont identiques ou différents, représentent des atomes d'hydrogène ou des radicaux alcoyle contenant 1 à 4 atomes de carbone, ou forment ensemble un radical alcoylène contenant 2 à 5 atomes de carbone, et

n est égal à 0 ou 1, ainsi que leurs formes isomères et leurs mélanges.

2. Un dérivé de la céphalosporine selon la revendication 1, caractérisé en ce que le symbole R est un radical trityle, le symbole R' est un radical benzhydryloxycarbonyle, le symbole Hal est un atome de brome et les symboles $R_a$, $R_b$, $R_c$ représentent chacun un atome d'hydrogène ou bien $R_a$ représente un

radical t.butoxycarbonyle et $R_b$ et $R_c$ représentent des radicaux alcoyle, ainsi que leurs formes isomères et leurs mélanges.

3. Un procédé de préparation d'un produit selon la revendication 1, pour lequel n égale 0, caractérisé en ce que l'on fait agir un agent d'halogénation sur une énamine de formule générale

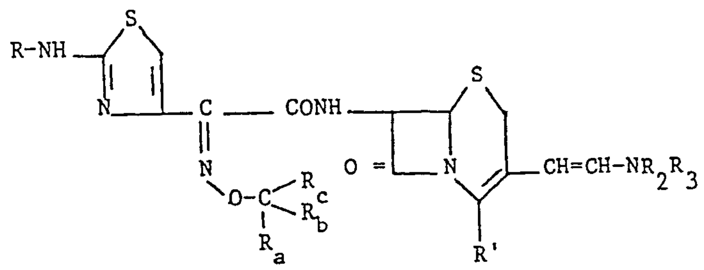

[dans laquelle R, R', $R_a$, $R_b$ et $R_c$ sont définis comme dans la revendication 1, et $R_2$ et $R_3$ qui sont identiques ou différents, représentent des radicaux alcoyle (éventuellement substitués par un radical alcoyloxy ou dialcoylamino) ou phényle, ou forment ensemble avec l'atome d'azote auquel ils sont rattachés, un hétérocycle à 5 ou 6 chaînons contenant éventuellement un autre hétéroatome choisi parmi l'azote, l'oxygène ou le soufre et éventuellement substitué par un radical alcoyle], puis hydrolyse le produit obtenu.

4. Un procédé de préparation d'un produit selon la revendication 1, pour lequel n égale 1, caractérisé en ce que l'on oxyde un produit selon la revendication 1, pour lequel n égale 0, par toute méthode connue qui n'altère pas le reste de la molécule.

**Revendication** (pour l'Etat contractant AT)

Procédé de préparation d'un dérivé de la céphalosporine de formule générale

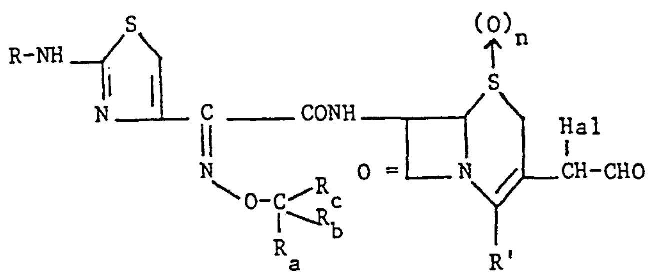

de forme syn, dans laquelle

le symbole R représente un radical protecteur d'amino,

le symbole R' représente un radical carboxy protégé,

le symbole Hal représente un atome d'halogène,

les symboles $R_a$, $R_b$ et $R_c$ représentent chacun un atome d'hydrogène, ou bien $R_a$ représente un radical carboxy protégé et, $R_b$ et $R_c$ qui sont identiques ou différents, représentent des atomes d'hydrogène ou des radicaux alcoyle contenant 1 à 4 atomes de carbone, ou forment ensemble un radical alcoylène contenant 2 à 5 atomes de carbone, et

n est égal à 0 ou 1, ainsi que leurs formes isomères et leurs mélanges caractérisé en ce que l'on fait agir un agent d'halogénation sur une énamine de formule générale

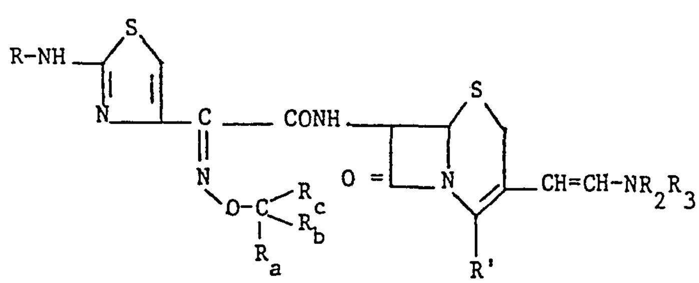

[dans laquelle R, R', $R_a$, $R_b$ et $R_c$ sont définis comme ci-dessus et, $R_2$ et $R_3$ qui sont identiques ou

différents, représentent des radicaux alcoyle (éventuellement substitués par un radical alcoyloxy ou dialcoylamino) ou phényle, ou forment ensemble avec l'atome d'azote auquel ils sont rattachés, un hétérocycle à 5 ou 6 chaînons contenant éventuellement un autre hétéroatome choisi parmi l'azote, l'oxygène ou le soufre et éventuellement substitué par un radical alcoyle], puis hydrolyse le produit obtenu, pour obtenir un produit pour lequel n égale 0, puis lorsque l'on veut obtenir un produit pour lequel n égale 1, on oxyde le produit correspondant pour lequel n égale 0, par toute méthode connue qui n'altère pas le reste de la molécule.

**Claims** (for the Contracting States : BE, CH, DE, FR, GB, IT, LI, LU, NL, SE)

1. A cephalosporin derivative, characterised in that it corresponds to the general formula

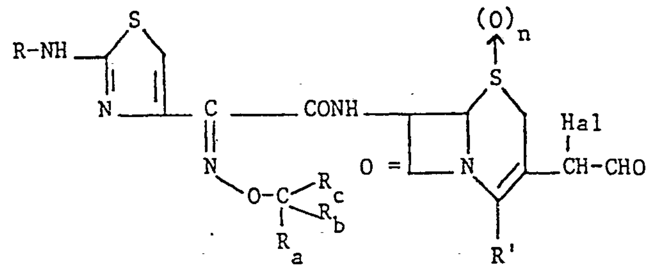

of syn form, in which
the symbol R denotes an amino-protecting radical,
the symbol R′ denotes a protected carboxy radical,
the symbol Hal denotes a halogen atom,
the symbols $R_a$, $R_b$ and $R_c$ each denote a hydrogen atom, or alternatively $R_a$ denotes a protected carboxy radical and $R_b$ and $R_c$, which are identical or different, denote hydrogen atoms or alkyl radicals containing 1 to 4 carbon atoms, or form together an alkylene radical containing 2 to 5 carbon atoms, and n equals 0 or 1, as well as isomeric forms thereof and mixtures thereof.

2. A cephalosporin derivative according to Claim 1, characterised in that the symbol R is a trityl radical, the symbol R′ is a benzhydryloxycarbonyl radical, the symbol Hal is a bromine atom and the symbols $R_a$, $R_b$ and $R_c$ each denote a hydrogen atom or alternatively $R_a$ denotes a t-butoxycarbonyl radical and $R_b$ and $R_c$ denote alkyl radicals, as well as isomeric forms thereof and mixtures thereof.

3. A process for preparing a product according to Claim 1, for which n equals 0, characterised in that a halogenating agent is reacted with an enamine of general formula

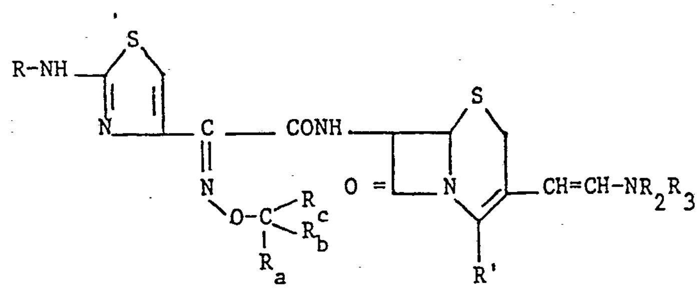

[in which R, R′, $R_a$, $R_b$ and $R_c$ are as defined in Claim 1 and $R_2$ and $R_3$, which are identical or different, denote alkyl radicals (optionally substituted with an alkyloxy or dialkylamino radical) or phenyl radicals, or form together with the nitrogen atom to which they are attached a 5- or 6-membered heterocycle optionally containing another hetero atom chosen from nitrogen, oxygen or sulfur, and optionally substituted with an alkyl radical], and the product obtained is then hydrolysed.

4. A process for preparing a product according to Claim 1, for which n equals 1, characterised in that a product according to Claim 1 for which n equals 0 is oxidised by any known method which does not alter the remainder of the molecule.

**Claim** (for the Contracting State AT)

Process for preparing a cephalosporin derivative of the general formula

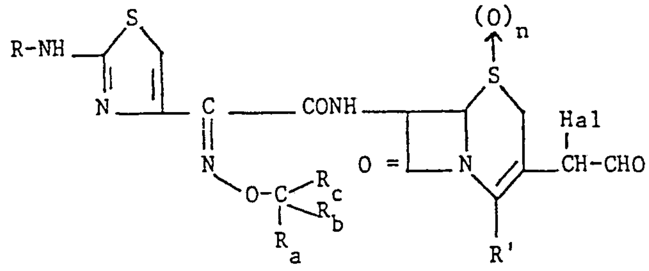

of syn form, in which
the symbol R denotes an amino-protecting radical,
the symbol R′ denotes a protected carboxy radical,
the symbol Hal denotes a halogen atom,
the symbols $R_a$, $R_b$ and $R_c$ each denote a hydrogen atom, or alternatively $R_a$ denotes a protected carboxy radical and $R_b$ and $R_c$, which are identical or different, denote hydrogen atoms or alkyl radicals containing 1 to 4 carbon atoms, or form together an alkylene radical containing 2 to 5 carbon atoms, and n equals 0 or 1, as well as isomeric forms thereof and mixtures thereof, characterised in that a halogenating agent is reacted with an enamine of general formula

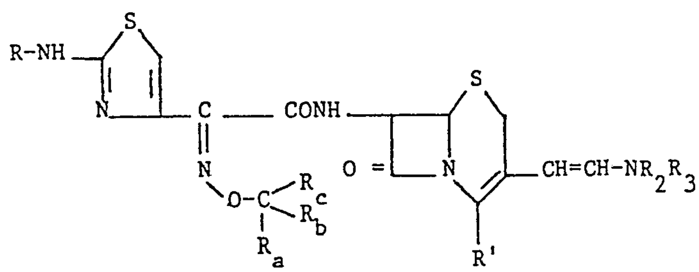

[in which R, R′, $R_a$, $R_b$ and $R_c$ are as defined above and $R_2$ and $R_3$, which are identical or different, denote alkyl radicals (optionally substituted with an alkyloxy or dialkylamino radical) or phenyl radicals, or form together with the nitrogen atom to which they are attached a 5- or 6-membered heterocycle optionally containing another hetero atom chosen from nitrogen, oxygen or sulphur, and optionally substituted with an alkyl radical], and the product obtained is then hydrolysed, to obtain a product for which n equals 0, and then, when it is desired to obtain a product for which n equals 1, the corresponding product for which n equals 0 is oxidised by any known method which does not alter the remainder of the molecule.

**Patentansprüche** (für die Vertragsstaaten : BE, CH, DE, FR, GB, IT, LI, LU, NL, SE)

1. Ein Derivat des Cephalosporins, dadurch gekennzeichnet, daß es der allgemeinen Formel

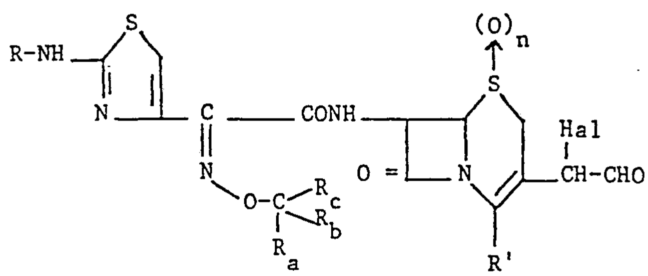

entspricht, der syn-Form, worin
das Symbol R einen Aminoschutzrest bedeutet,
das Symbol R′ einen geschützten Carboxyrest bedeutet,
das Symbol Hal ein Halogenatom bedeutet,
die Symbole $R_a$, $R_b$ und $R_c$ jeweils ein Wasserstoffatom bedeuten, oder aber $R_a$ bedeutet einen geschützen Carboxyrest, und $R_b$ und $R_c$, die identisch oder verschieden sind, bedeuten Wasserstoffatome oder Alkylreste mit 1 bis 4 Kohlenstoffatomen oder bilden zusammen einen Alkylenrest mit 2 bis 5 Kohlenstoffatomen,
und n = 0 oder 1, sowie ihre isomeren Formen und ihre Gemische.

2. Ein Derivat des Cephalosporins gemäß Anspruch 1, dadurch gekennzeichnet, daß das Symbol R ein Tritylrest ist, das Symbol R' ein Benzhydryloxycarbonylrest ist, das Symbol Hal ein Bromatom ist, und die Symbole $R_a$, $R_b$ und $R_c$ jeweils ein Wasserstoffatom bedeuten, oder aber $R_a$ bedeutet einen tert.-Butoxycarbonylrest, und $R_b$ und $R_c$ bedeuten Alkylreste, sowie ihre isomeren Formen und ihre Gemische.

3. Verfahren zur Herstellung eines Produkts gemäß Anspruch 1, wöfur n = 0, dadurch gekennzeichnet, daß man ein Halogenierungsmittel auf ein Enamin der allgemeinen Formel

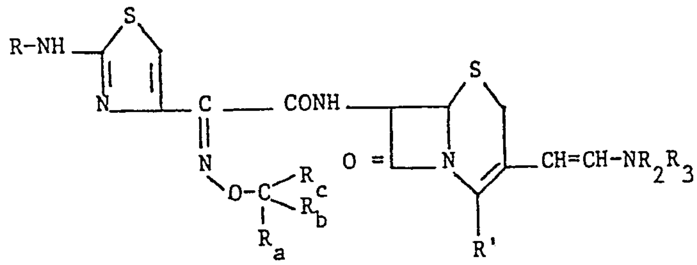

[worin R, R', $R_a$, $R_b$ und $R_c$ wie in Anspruch 1 definiert sind, und $R_2$ und $R_3$, die identisch oder verschieden sind, Alkylreste (gegebenenfalls substituiert durch einen Alkyloxy- oder Dialkylaminorest) oder Phenyl bedeuten, oder zusammen mit dem Stickstoffatom, an das sie gebunden sind, einen Heterocyclus mit 5 oder 6 Gliedern bilden, gegebenenfalls enthaltend ein weiteres Heteroatom, ausgewählt unter Stickstoff, Sauerstoff oder Schwefel und gegebenenfalls substituiert durch einen Alkylrest] einwirken läßt, und daß man dann das erhaltene Produkt hydrolysiert.

4. Verfahren zur Herstellung eines Produkts gemäß Anspruch 1, wobei n = 1, dadurch gekennzeichnet, daß man ein Produkt gemäß Anspruch 1, wobei n = 0, nach jeder bekannten Methode, welche den Rest des Moleküls nicht verändert, oxydiert.

**Patentanspruch** (für den Vertragsstaat AT)

Verfahren zur Herstellung eines Cephalosporinderivats der allgemeinen Formel

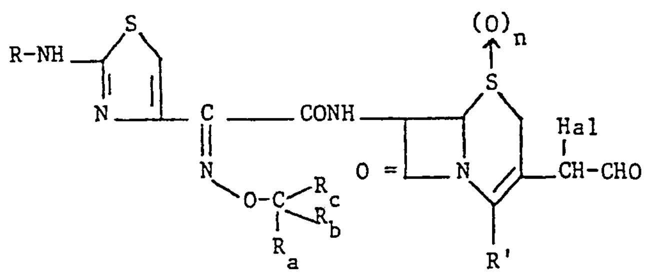

in syn-Form, in welcher

das Symbol R eine Aminoschutzgruppe darstellt,

das Symbol R' einen geschützten Carboxyrest dartstellt,

das Symbol Hal ein Halogenatom darstellt,

die Symbole $R_a$, $R_b$ und $R_c$ jeweils ein Wasserstoffatom darstellen oder $R_a$ einen geschützten Carboxyrest darstellt und $R_b$ und $R_c$, die gleich oder voneinander verschieden sind, Wasserstoffatome oder Alkylreste mit 1 bis 4 Kohlenstoffatomen darstellen oder zusammen einen Alkylenrest mit 2 bis 5 Kohlenstoffatomen bilden,

und n gleich 0 oder 1 ist, sowie von deren isomeren Formen und ihren Mischungen, dadurch gekennzeichnet, daß man ein Halogenierungsmittel auf ein Enamin der allgemeinen Formel

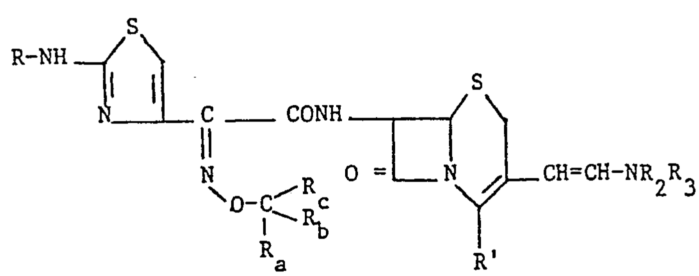

[in welcher R, R', $R_a$, $R_b$ und $R_c$ die obige Bedeutung haben und $R_2$ und $R_3$, die gleich oder voneinander verschieden sind, Alkylreste (gegebenenfalls substituiert durch einen Alkyloxy- oder Dialkylaminorest) oder Phenylreste darstellen oder zusammen mit dem Stickstoffatom, an das sie gebunden sind, einen Heterocyclus mit 5 oder 6 Gliedern bilden, der gegebenenfalls ein weiteres Heteroatom, ausgewählt aus Stickstoff, Sauerstoff oder Schwefel, enthält und gegebenenfalls durch einen Alkylrest substituiert ist] einwirken läßt, dann die erhaltene Verbindung hydrolysiert unter Bildung einer Verbindung erhalten will, worin n gleich 1 ist, die entsprechende Verbindung, worin n gleich 0 ist, nach irgendeiner bekannten Methode oxidiert, wobei der Rest des Moleküls nicht verändert wird.